(19) Europäisches Patentamt
European Patent Office
Office européen des brevets

(11) Numéro de publication : **0 445 190 B1**

(12) # FASCICULE DE BREVET EUROPEEN

(45) Date de publication du fascicule du brevet : **15.09.93 Bulletin 93/37**

(51) Int. Cl.$^5$ : **A61K 49/02,** C07F 13/00

(21) Numéro de dépôt : **89913235.1**

(22) Date de dépôt : **24.11.89**

(86) Numéro de dépôt international : **PCT/FR89/00608**

(87) Numéro de publication internationale : **WO 90/06137 14.06.90 Gazette 90/14**

(54) **PRODUIT RADIOPHARMACEUTIQUE AYANT NOTAMMENT UN TROPISME CARDIAQUE COMPORTANT UN COMPLEXE NITRURO D'UN METAL DE TRANSITION, ET SON PROCEDE DE PREPARATION.**

(30) Priorité : **25.11.88 FR 8815415**
**12.06.89 FR 8907731**

(43) Date de publication de la demande : **11.09.91 Bulletin 91/37**

(45) Mention de la délivrance du brevet : **15.09.93 Bulletin 93/37**

(84) Etats contractants désignés : **AT BE CH DE ES FR GB IT LI LU NL SE**

(56) Documents cités :
**WO-A-85/03063**
**Intern. J. of Radiation/Applications Instrumentation part A, vol. 38, no. 8, 1987, GB pp. 665-668; Ballinger J.R.**
**Intern. J. of Applied Radiation and Isotopes, vol. 36, no. 2, Febr. 1985, Oxford, GB pp. 133-139; Baldas J. et al.**

(73) Titulaire : **CIS BIO INTERNATIONAL**
**RN 306**
**F-91400 Saclay (FR)**

(72) Inventeur : **PASQUALINI, Roberto**
**223, avenue Victor-Hugo**
**F-92140 Clamart (FR)**
Inventeur : **MAGON, Luciano**
**Via Pietro Selvatico, 86**
**I-35100 Padova (IT)**
Inventeur : **BARDY, André**
**48, les Genêts**
**F-92420 Morangis (FR)**
Inventeur : **DUATTI, Adriano**
**Via Galvana, 34**
**I-44040 Chiesuol Fosso (IT)**
Inventeur : **MARCHI, Andrea**
**Via Arianuova, 75**
**I-44100 Ferrara (IT)**

(74) Mandataire : **Des Termes, Monique et al**
**Société Brevatome 25, rue de Ponthieu**
**F-75008 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet un produit radiopharmaceutique ayant notamment un tropisme cardiaque, qui comprend un complexe nitruro d'un métal de transition comportant une partie M ≡ N, dans laquelle M représente le métal de transition. Elle s'applique plus particulièrement aux produits radiopharmaceutiques à tropisme cardiaque.

On précise que l'on entend par métal de transition, un métal dont la couche d est partiellement remplie dans le degré d'oxydation usuel de ce métal. Il s'agit des éléments remplissant les périodes III à XII du tableau périodique des éléments à dix-huit colonnes.

A titre d'exemple de tels métaux, on peut citer Tc, Ru, Co, Pt, Fe, Os, Ir, W, Re, Cr, Mo, Mn, Ni, Rh, Pd, Nb et Ta.

Des complexes nitruro de technétium ont été décrits par J. Baldas et Col. dans les documents suivants : demande de brevet internationale W0-85/03 063, J. Chem. Soc. Dalton Trans., 1981, pages 1798-1801, et le livre "Technetium in Chemistry and Nuclear Medicine", Ed. M. Nicolini, G. Bandoli, U. Mazzi, Cortine Int. Verone, 1986, pages 103 à 108.

Dans ces documents, on décrit la préparation de complexes nitruro de technétium par réaction de substitution sur $^{99m}TcNCl_4$ et il est indiqué que ces complexes peuvent être utilisés comme produits radiopharmaceutiques, mais ces documents ne donnent aucun résultat probant sur la fixation de ces complexes dans le corps, et ne donnent donc aucune indication sur leurs tropismes, vis-à-vis de certains organes, notamment du coeur.

Parmi les produits radiopharmaceutiques ayant un tropisme cardiaque, on connaît des complexes de technétium contenant comme ligand des isonitriles substitués par un éther, comme il est décrit dans la demande de brevet européen EP-A- 0233368, et des complexes de technétium à base de dioxime comme il est décrit dans le brevet européen EP-A-0268801. Ces complexes sont formés à partir de ligands difficiles à synthétiser.

Aussi, des recherches ont été développées pour trouver d'autres produits radiopharmaceutiques présentant des propriétés satisfaisantes pour une utilisation comme produits de diagnostic ou de thérapie, en particulier des produits radiopharmaceutiques à tropisme cardiaque, par exemple pour la scintigraphie du myocarde.

La présente invention a précisément pour objet un produit radiopharmaceutique qui comprend un complexe d'un métal de transition répondant à la formule :

$$(M \equiv N)\ L^1L^2$$

dans laquelle M est un métal de transition et $L^1$ et $L^2$ qui peuvent être identiques ou différents, répondent à la formule :

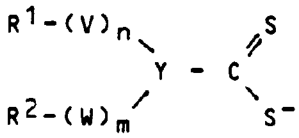

(II)

dans laquelle V et W qui peuvent être identiques ou différents, représentent O, S ou Se, n et m qui peuvent être identiques ou différents, sont égaux à 0 ou à 1, Y représente N, P ou As, et $R^1$ et $R^2$ qui peuvent être identiques ou différents, représentent un radical alkyle linéaire ou ramifié de 1 à 10 atomes de carbone, non substitué ou substitué par des groupements $-O-R^3$, $OOC-R^3$, $OCNR^4R^5$ ou $-NR^4R^5$ dans lesquels $R^3$ est un radical alkyle linéaire ou ramifié de 1 à 5 atomes de carbone et $R^4$ et $R^5$ qui peuvent être identiques ou différents, son des atomes d'hydrogène ou des radicaux alkyle linéaires ou ramifiés de 1 à 5 atomes de carbone, ou dans laquelle $R^1$ et $R^2$ forment ensemble un cycle hydrocarboné contenant éventuellement un ou plusieurs hétéroatomes.

Les produits radiopharmaceutiques comprenant les complexes de métaux de transition répondant à la formule donnée ci-dessus présentent notamment un tropisme cardiaque, ce qui les rend intéressants comme produits de diagnostic ou de thérapie pour le coeur.

Dans les produits radiopharmaceutiques de l'invention, le complexe nitruro de métal de transition peut être de différents types.

Ainsi, selon un premier mode de réalisation de l'invention, dans la formule donnée ci-dessus, Y représente N, m et n sont égaux à O, $L^1$ et $L^2$ sont identiques et $R^1$ et $R^2$ sont des radicaux alkyle non substitués.

Dans ce premier mode de réalisation de l'invention $R^1$ et $R^2$ sont de préférence également identiques.

A titre d'exemple, $L^1$ et $L^2$ peuvent répondre aux formules suivantes :

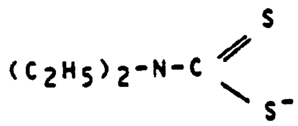

(III)

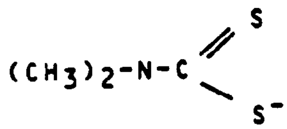

(IV)

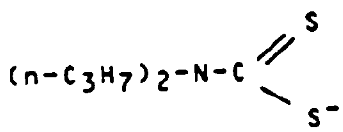

(V)

Selon un second mode de réalisation de l'invention, Y représente N, m et n sont égaux à O, $L^1$ et $L^2$ sont identiques et l'un au moins des $R^1$ et $R^2$ représente un radical alkoxyalkyle.

A titre d'exemple, $L^1$ et $L^2$ peuvent répondre aux formules suivantes :

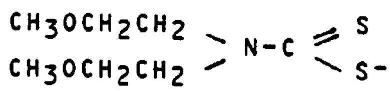

(IV)

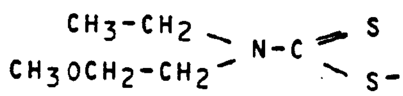

(VII)

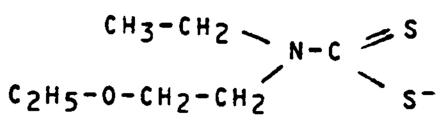

(VIII)

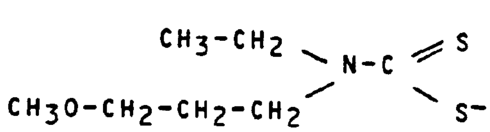

(IX)

Selon un troisième mode de réalisation de l'invention le complexe de métal de transition répond à la formule :

$$(M{\equiv}N)L^1L^2$$

dans laquelle M est un métal de transition et $L^1$ et $L^2$ répondent à la formule :

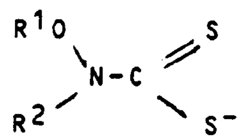

dans laquelle $R^1$ et $R^2$ ont la signification donnée dans la revendication 1.

A titre d'exemples de tels complexes, on peut citer ceux pour lesquels $R^1$ représente $CH_3$ et $R^2$ représente $CH_3$- ou $CH_3$-$CH_2$-. ou pour lesquels $R^1$ représente $CH_3$-$CH_2$- et $R^2$ représente $CH_3$- ou $CH_3$-$CH_2$-.
Dans les complexes de l'invention, le métal de transition utilisé dépend en particulier de l'application du produit radiopharmaceutique.

Ainsi, lorsqu'on veut utiliser le produit pour le diagnostic, on utilise un métal de transition radioactif, ayant une période relativement courte, par exemple le technétium $^{99m}$.

Dans le cas où l'on veut utiliser le produit radiopharmaceutique pour la thérapie, on utilise un métal de transition émettant un rayonnement efficace pour la thérapie, et ayant une durée de vie plus longue tel que le rhénium, par exemple Re-186 ou Re-188.

Les complexes nitruro de technétium utilisés dans l'invention, peuvent être préparés par le procédé de Baldas. Toutefois, on préfère généralement les préparer par un procédé plus simple, facile à mettre en oeuvre dans un service hospitalier et conduisant à des rendements élevés.

Ce procédé comprend les étapes successives suivantes :

1°) faire réagir un composé oxygéné d'un métal de transition M avec :

a) un premier ligand choisi dans le groupe des phosphines et polyphosphines aliphatiques et aromatiques, substituées ou non substituées, et

b) un second réactif choisi parmi les azotures de métal alcalin et d'ammonium et les ligands azotés comportant un motif >N-N< dans lesquels les N sont reliés à des atomes d'hydrogène et/ou à des groupements organiques monovalents par l'intermédiaire d'un atome de carbone, ou dans lequel l'un des N est relié à l'atome de carbone d'un groupe organique bivalent par l'intermédiaire d'une double liaison et l'autre N est relié à des atomes d'hydrogène et/ou à des groupements organiques monovalents par l'intermédiaire d'un atome de carbone, et

2°) faire réagir le produit intermédiaire obtenu dans la première étape avec un composé répondant à la formule :

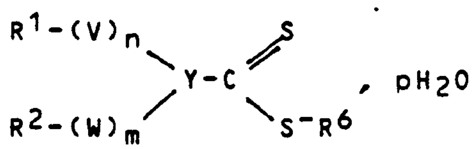 (X)

dans laquelle $R^1$, $R^2$, V, W, n, m et Y ont la signification donnée ci-dessus,
$R^6$ est un ion de métal alcalin, $H^+$ ou $NH_4^+$, et p est égal à 0 ou est un nombre entier allant de 1 à 5.

Lorsque l'on met en oeuvre ce procédé en utilisant comme métal de transition du technétium, le composé oxygéné du métal de transition peut être du pertechnétate de métal alcalin ou d'ammonium. Dans le cas où le métal de transition est le rhénium, on peut utiliser un perrhénate de métal alcalin ou d'ammonium.

Dans la première étape du procédé, on prépare ainsi un premier complexe nitruro de technétium que l'on fait réagir ensuite avec le composé de formule (X) pour échanger le premier et le deuxième ligands par ce composé.

Pour réaliser la réaction, on peut introduire aseptiquement le premier ligand et soit l'azoture de métal alcalin ou d'ammonium, soit le ligand azoté, dans un récipient, puis ajouter la quantité requise de composé oxygéné de métal de transition, par exemple de pertechnétate de technétium $^{99m}$, après avoir ajusté le pH à une valeur appropriée par addition d'acide ou de base. On peut ensuite effectuer la réaction à la température ambiante ou à une température supérieure allant de 50 à 100°C. La température et le pH utilisés dépendent en particulier du second ligand azoté. Généralement on opère entre pH 2 et 7.

Dans la première étape, on peut utiliser le premier et le second ligands sous la forme de solutions aqueuses, alcooliques ou hydroalcooliques et ajouter simplement ces solutions au composé oxygéné du métal de transition.

Dans la deuxième étape, on fait réagir le produit obtenu dans la première étape avec le composé de formule (X) en solution aqueuse, généralement à un pH supérieur à 7, par exemple dans un tampon carbonate-bicarbonate de sodium.

Dans cette deuxième étape, on peut aussi utiliser une solution alcoolique ou hydroalcoolique du composé (X).

Le premier ligand qui permet d'obtenir la formation d'un complexe nitruro est un ligand organique à atome de phosphore donneur d'électrons choisi parmi les phosphines et les polyphosphines aliphatiques et aromatiques, substituées ou non substituées.

Les phosphines utilisables peuvent répondre à la formule :

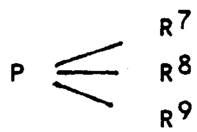

dans laquelle $R^7$, $R^8$ et $R^9$ qui peuvent être identiques ou différents, représentent un atome d'hydrogène, un radical alkyle, un radical aryle, un radical alcoxy ou un radical alkyle ou aryle substitué par un groupement choisi parmi les radicaux amino, amido, cyano et sulfonate.

A titre d'exemple de phosphines de ce type, on peut citer la triphénylphosphine, la triphénylphosphine trisulfonée, la diéthylphénylphosphine, la triéthylphosphine, la triméthylphosphine et la tris(cyanoéthyl-2)phosphine $P(CH_2-CH_2CN)_3$.

Dans la première étape, on peut utiliser comme second réactif, soit un azoture de métal alcalin ou d'ammonium, par exemple de l'azoture de sodium, soit un ligand azoté comportant le motif >N-N< comme dans l'hydrazine et ses dérivés. On peut utiliser de nombreux ligands azotés de ce type. Généralement, on préfère utiliser comme ligand azoté, l'acide dithiocarbazique ou un dérivé de celui-ci.

Ainsi, le second ligand azoté peut être l'acide dithiocarbazique ou un dérivé de celui-ci répondant à la formule :

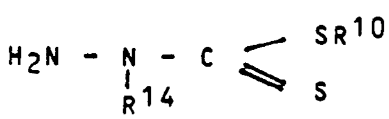

dans laquelle $R^{10}$ représente un atome d'hydrogène, un radical alkyle ou un radical aryle, et $R^{14}$ représente un atome d'hydrogène, un radical alkyle, un radical aryle, un radical alcoxy, un radical alkyle substitué par au moins un groupement choisi parmi les radicaux hydroxy, carboxy, amino, amido et mercapto, ou un radical aryle substitué par au moins un groupement choisi parmi les atomes d'halogène et les radicaux alcoxy, hydroxy, amino, mercapto et amino substitué par au moins un radical alkyle.

Il peut également être un produit de condensation obtenu par réaction de l'acide dithiocarbazique avec une cétone ou un aldéhyde aliphatique de formule $R^{15}-CO-R^{16}$. Dans ce cas, il répond à la formule :

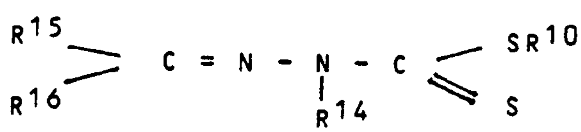

dans laquelle $R^{10}$ représente un atome d'hydrogène, un radical alkyle ou un radical aryle ; $R^{14}$ représente un atome d'hydrogène, un radical alkyle, un radical aryle, un radical alcoxy, un radical alkyle substitué par au moins un groupement choisi parmi les radicaux hydroxy, carboxy, amino, amido et mercapto, ou un radical aryle substitué par au moins un groupement choisi parmi les atomes d'halogène et les radicaux alkoxy, hydroxy, amino, mercapto et amino substitué par au moins un radical alkyle ; et $R^{15}$ et $R^{16}$ qui peuvent être identiques ou différents représentent un atome d'hydrogène, un radical alkyle ou un radical alkyle substitué par au moins un groupement choisi parmi les radicaux hydroxy, carboxy, amino, amido et mercapto.

Le dérivé d'acide dithiocarbazique utilisé comme second ligand peut aussi être le produit de condensation de l'acide dithiocarbazique avec une cétone ou un aldéhyde aromatique. Dans ce cas, le dérivé répond à la formule :

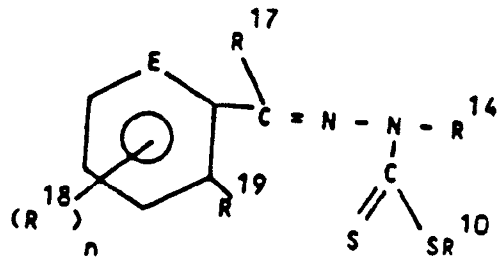

dans laquelle $R^{10}$ représente un atome d'hydrogène, un radical alkyle ou un radical aryle ; $R^{14}$ représente un atome d'hydrogène, un radical alkyle, un radical aryle, un radical alcoxy, un radical alkyle substitué par au moins un groupement choisi parmi les radicaux hydroxy, carboxy, amino, amido et mercapto ou un radical aryle substitué par au moins un groupement choisi parmi les atomes d'halogène et les radicaux alkoxy, hydroxy, amino, mercapto et amino substitué par au moins un radical alkyle ; $R^{17}$ représente un atome d'hydrogène, un radical alkyle, un radical alkyle substitué par au moins un groupement choisi parmi les radicaux hydroxy, carboxy, amino, amido et mercapto, $R^{18}$ représente un atome d'hydrogène, un atome d'halogène, un radical alcoxy, un radical amino, ou un radical amino substitué par au moins un groupement alkyle, $R^{19}$ représente un atome d'hydrogène, un radical hydroxy ou un radical mercapto, E représente un atome de carbone ou un atome d'azote, et n est un nombre entier allant de 1 à 4, ou dans laquelle n est égal à 2 et les deux $R^{18}$ sont voisins et forment ensemble un cycle aromatique.

On peut également utiliser comme second ligand le produit obtenu par condensation de l'acide dithiocarbazique avec une cétone comportant un hétérocycle à 5 maillons. Dans ce cas, le second ligand répond à la formule :

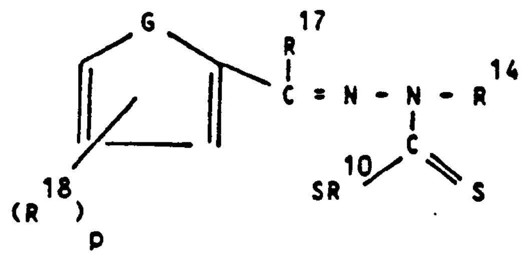

dans laquelle $R^{10}$ représente un atome d'hydrogène, un radical alkyle ou un radical aryle ; $R^{14}$ représente un atome d'hydrogène, un radical alkyle, un radical aryle, un radical alkoxy, un radical alkyle substitué par au moins un groupement choisi parmi les radicaux hydroxy, carboxy, amino, amido et mercapto, ou un radical aryle substitué par au moins un groupement choisi parmi les atomes d'halogène et les radicaux alkoxy, hydroxy, amino, mercapto et amino substitué par au moins un radical alkyle ; $R^{17}$ représente un atome d'hydrogène, un radical alkyle, un radical alkyle substitué par au moins un groupement choisi parmi les radicaux hydroxy, carboxy, amino, amido et mercapto, $R^{18}$ représente un atome d'hydrogène, un atome d'halogène, un radical alcoxy, un radical amino, ou un radical amino substitué par au moins un groupement alkyle, G est S ou 0, et p est 1, 2 ou 3.

A titre d'exemple de seconds ligands azotés susceptibles d'être utilisés, on peut citer, le S-méthyl-bêta-N(2-hydroxyphényl)méthylène dithiocarbazate, le S-méthyldithiocarbazate, le S-méthyl-N-méthyl-dithiocarbazate, l'alpha-N-méthyl-S-méthyl-bêta-N-pyridylméthylène dithiocarbazate, et le alpha-N-méthyl-S-méthyl-bêta-N(2-hydroxyphényl) méthylène dithiocarbazate.

Lorsque le produit radiopharmaceutique de l'invention est destiné au diagnostic, il est généralement nécessaire de le préparer au moment de l'utilisation.

Aussi, l'invention a également pour objet une trousse pour la préparation d'un produit radiopharmaceutique à tropisme cardiaque, qui comprend :

- un premier flacon contenant une phosphine ;
- un second flacon contenant de l'azoture de sodium, de l'acide dithiocarbazique ou un dérivé de celui-ci, et,
- un troisième flacon contenant un composé répondant à la formule :

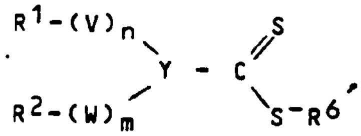

, $pH_2O$ (X)

dans laquelle $R^1$, $R^2$, V, W, n, m et Y ont la signification donnée ci-dessus, $R^6$ est un ion de métal alcalin, $H^+$ ou $NH_4^+$, et p est égal à 0 ou est un nombre entier allant de 1 à 5.

Avec cette trousse, on peut préparer directement le produit radiopharmaceutique voulu, dans un service hospitalier de médecine nucléaire, en mélangeant le contenu des deux premiers flacons avec une solution du composé oxygéné de métal de transition, par exemple une solution de pertechnétate de métal alcalin ou d'ammonium, puis ajouter au produit ainsi obtenu le contenu du troisième flacon.

Les produits présents respectivement dans les premier, second et troisième flacons peuvent être sous forme liquide ou sous forme lyophilisée.

Dans certains cas, on peut aussi mélanger le contenu des deux premiers flacons avant utilisation. Dans ce cas, la trousse comprendra uniquement un premier flacon contenant une phosphine et le second réactif constitué soit par l'azoture de sodium, soit par l'acide dithiocarbazique ou un dérivé de celui-ci, et un second flacon contenant le composé de formule VII donnée ci-dessus.

Etant donné que les produits sont destinés à une injection intraveineuse à des êtres vivants, on doit utiliser des conditions appropriées de fabrication et de mise en oeuvre pour obtenir des solutions convenablement stériles et apyrogènes.

Pour préparer les solutions, on peut utiliser de l'eau stérile et apyrogène ou des solutions alcooliques ou hydroalcooliques, stériles et apyrogènes, et effectuer un stockage des solutions sous azote.

Pour préparer des compositions lyophilisées, on soumet à une lyophilisation dans un équipement classique des solutions obtenues dans les mêmes conditions que précédemment.

Les produits radiopharmaceutiques de l'invention peuvent être utilisés en particulier pour la scintigraphie du myocarde.

Dans ce cas, après préparation du complexe nitruro de technétium, on injecte celui-ci au patient à examiner, et on procède ensuite à un examen du coeur par scintigraphie.

Pour l'injection du produit, les quantités des différents ligands sont telles qu'elles correspondent sensiblement à la stoechiométrie des complexes à obtenir. La quantité finale injectée dépend en particulier des ligands utilisés et de leur toxicité.

Généralement, on obtient des résultats satisfaisants en utilisant des quantités totales de ligands allant de 0,05 à 0,40mg/kg de poids corporel.

La dose totale de métal de transition par exemple de technétium, se situe généralement dans la gamme de 185 à 740Mbq (5 à 20millicuries).

Après l'administration du complexe nitruro de métal de transition, on peut effectuer un examen satisfaisant dans un délai de 0,5 à 3h en obtenant un bon contraste, des images nettes et une bonne détection des lésions.

D'autres caractéristiques et avantages de l'invention apparaîtront mieux à la lecture des exemples suivants.

**Exemple 1 :** Préparation du complexe nitruro-bis(diéthyldithiocarbamate)$^{99m}$Tc(V)(TcNDEDC).

a) Préparation du produit intermédiaire.

Dans un flacon type pénicilline, on introduit 0,4ml d'une solution contenant $2.10^{-2}$mol/l (2,5mg/ml) de S-méthyldithiocarbazate dans l'alcool éthylique, puis 0,2ml d'une solution à $2.10^{-2}$mol/l (5mg/ml) de triphénylphosphine dans l'alcool éthylique et 0,1ml d'acide chlorhydrique 1N. On ajoute ensuite 0,5 à 1ml d'une solution de pertechnétate de sodium ($Tc^{99m}$) et on effectue la réaction à 80°C pendant 30 minutes ou à 100°C pendant 15 minutes.

b) Préparation du complexe final.

Au contenu du flacon obtenu dans l'étape a), on ajoute 0,1ml de solution de NaOH 1N et 0,5ml d'une solution contenant 0,18mol/l de diéthyldithiocarbamate de sodium trihydraté (40mg/ml) dans un tampon carbonate-bicarbonate de sodium à 0,5 $mol/l^{-1}$ à pH 9,0.

On effectue la réaction pendant 15 minutes à 100°C, pendant 30 minutes à 80°C, ou pendant 60 minutes à la température ambiante.

La pureté radiochimique du complexe obtenu est testée en effectuant une chromatographie sur couche mince en utilisant un gel de silice et le toluène comme solvant.

Le complexe obtenu a un Rf de 0,3 à 0,4.

La pureté radiochimique est supérieure ou égale à 93%.

**Exemple 2 :** Préparation du complexe nitruro-bis(diéthyldithiocarbamate)$^{99m}$Tc(V) (TcNDEDC).

a) Préparation du produit intermédiaire.

Dans un flacon type pénicilline, on introduit 0,2ml d'une solution contenant 7,7.$10^{-2}$mol/l (5,0mg/ml)d'azoture de sodium dans de l'eau, puis 0,2ml d'une solution à 2.$10^{-2}$mol/l (5mg/ml) de triphénylphosphine dans l'alcool éthylique et 0,1ml d'acide chlorhydrique 1N. On ajoute ensuite 0,5 à 1ml d'une solution de pertechnétate de sodium (Tc-99m) et on effectue la réaction à 80°C pendant 30 minutes ou à 100°C pendant 15 minutes.

b) Préparation du produit final.

Au contenu du flacon obtenu dans l'étape a), on ajoute comme dans l'exemple 1, 0,1ml d'une solution de NaOH 1N et 0,5ml d'une solution contenant 0,18mol/l (40mg/ml) de diéthyldithiocarbamate de sodium trihydraté dans un tampon carbonate/bicarbonate de sodium à 0,5mol/l et à pH 9. On effectue la réaction comme dans l'exemple 1.

On teste également la pureté radiochimique du produit par chromatographie sur couche mince et on obtient des résultats identiques à ceux de l'exemple 1.

**Exemple 3 :** Préparation du complexe nitruro-bis (diéthyldithiocarbamate) $^{99m}$Tc(V) (TcNDEDC).

a) Préparation du produit intermédiaire.

Dans un flacon type pénicilline, on introduit 0,2ml d'une solution contenant 7.7.$10^{-2}$mol/l (5mg/ml) d'azoture de sodium dans l'eau, puis 0,4ml d'une solution contenant 1.$10^{-2}$mol/l (2mg/ml) de tris(cyanoéthyl-2)phosphine dans l'eau et 0,1ml d'acide chlorhydrique 1N.

On ajoute ensuite 0,5 à 5ml d'une solution de pertechnetate de sodium ($^{99m}$Tc) et on effectue la réaction à 80°C pendant 30 minutes ou à 100°C pendant 15 minutes. (Préparation sans alcool)

b) Préparation du complexe final

On suit le même mode opératoire que dans l'exemple 1, paragraphe b) pour préparer le complexe TcNDEDC à partir du produit intermédiaire obtenu précédemment.

**Exemple 4 :** Préparation du complexe nitruro-bis(diéthyldithiocarbamate)$^{99m}$Tc(V) (TcNDEDC).

a) Préparation du produit intermédiaire.

Dans un flacon type pénicilline, on introduit 0,2ml d'une solution contenant 7.7.$10^{-2}$mol/l (5mg/ml) d'azoture de sodium dans l'eau, puis 0,4ml d'une solution contenant 1.$10^{-2}$mol/l de tris(cyanoéthyl-2)phosphine.

On ajoute ensuite 0,5 à 5ml d'une solution de pertechnétate de sodium ($^{99m}$Tc) et on effectue la réaction à 80°C pendant 30 minutes ou à 100°C pendant 15 minutes.

Cette opération a lieu à un pH proche de 7.

b) Préparation du produit final.

Au contenu du flacon obtenu dans l'étape a), on ajoute 0,5ml d'une solution contenant 0,18mol/l (40mg/ml) de diéthyldithiocarbamate de sodium trihydraté dans un tampon carbonate/bicarbonate de sodium à 0,5mol/l et à pH9. On effectue la réaction comme dans l'exemple 1.

**Exemple 5 :** Préparation du complexe nitruro-bis(diméthyldithiocarbamate)$^{99m}$Tc (V) (TcNDMDC).

On suit le même mode opératoire que dans l'exemple 1 en remplaçant dans la dernière étape le diéthyldithiocarbamate par 0,5ml d'une solution contenant 0,18mol/l de diméthyldithiocarbamate de sodium dihydraté

(30mg/ml) dans le même tampon. On obtient ainsi le complexe de technétium TcNDMDC avec une pureté radiochimique équivalente à celle de l'exemple 1.

**Exemple 6** : Préparation du complexe nitruro-bis(di-n-propyldithiocarbamate)$^{99m}$Tc (V) (TcNDPDC).

On suit le même mode opératoire que dans l'exemple 1 en remplaçant le diéthyldithiocarbamate par 0,5ml d'une solution contenant 0,18mol/l (40,7mg/ml)de di-n-propyldithiocarbamate de sodium sesquihydraté dans un mélange du tampon carbonate-bicarbonate de sodium à 0,5mol/l et à pH 9 et d'alcool éthylique dans un rapport en volume 7:3.

On obtient ainsi le complexe de technétium TcNDPDC présentant une pureté radiochimique équivalente à celle de l'exemple 1.

**Exemples 7 à 9.**

Dans ces exemples, on teste les propriétés des complexes obtenus dans les exemples 1, 5 et 6, en déterminant leur biodistribution chez des rats mâles de la race Sprague Dawley, pesant 200±20g.

Dans ce cas, on injecte aux rats, anesthésiés avec du pentobarbital de sodium, une dose correspondant à 15μmol/kg de masse corporelle de ligand myotrope, ce qui correspond à une dose de rayonnement de 1 à 2,5μCi.

5 minutes, 30 minutes, ou 60 minutes après l'injection du produit, on sacrifie les rats, on prélève leurs organes et on détermine la radioactivité présente dans chacun des organes.

Les résultats obtenus sont donnés dans le tableau 1 qui suit, et exprimés en pourcentage de la radioactivité injectée retrouvée dans l'organe, après prélèvement et comptage.

Les valeurs données dans chaque case du tableau représentent la valeur moyenne et les deux valeurs extrêmes.

Au vu de ce tableau, on constate que ces complexes présentent un bon tropisme cardiaque.

TABLEAU 1

| Temps entre injection I.V. et sacrifice / organes entiers | TcNDMDC $(CH_3)_2$N-C 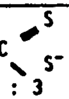 nombre d'animaux : 3 | | | TcNDEDC $(C_2H_5)_2$N-C 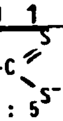 nombre d'animaux : 5 | | | TNDPDC $(C_3H_7)_2$-N-C 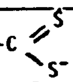 nombre d'animaux : 5 | | |
|---|---|---|---|---|---|---|---|---|---|
| | 5 min | 30 min | 60 min | 5 min | 30 min | 60 min | 5 min | 30 min | 60 min |
| Foie | 17,5<br>16,6 - 18,1 | 18,2<br>16,7 - 20,0 | 27,5<br>27,6 - 28,5 | 20,2<br>14,9 - 22,1 | 24,1<br>23,2 - 26,1 | 25<br>24,3 - 26,1 | 27,2<br>23,1 - 32,3 | 33,2<br>29,8 - 35,2 | 28,5<br>22,3 - 33,4 |
| Reins | 5,2<br>5,1 - 5,3 | 1,9<br>1,8 - 2,1 | 1,8<br>1,7 - 2,2 | 4,9<br>4,6 - 5,2 | 3,5<br>3,2 - 4,1 | 3,0<br>2,8 - 3,2 | 2,9<br>2,4 - 3,6 | 2,6<br>2,0 - 3,3 | 2,1<br>1,6 - 2,6 |
| Poumons | 4,4<br>3,9 - 4,6 | 6,0<br>4,5 - 7,5 | 6,5<br>4,5 - 11,5 | 9,2<br>8,5 - 9,5 | 1,8<br>1,6 - 1,9 | 1,2<br>1,3 - 1,4 | 2,6<br>2,1 - 3,4 | 1,1<br>1,0 - 1,3 | 0,6<br>0,4 - 0,9 |
| Cerveau | 0,52<br>0,48 - 0,58 | 0,18<br>0,17 - 0,19 | 0,15<br>0,14 - 0,16 | 0,7<br>0,7 - 0,7 | 0,2<br>0,2 - 0,2 | 0,1<br>0,1 - 0,1 | 0,2<br>0,1 - 0,2 | 0,2<br>0,2 - 0,2 | 0,2<br>0,1 - 0,2 |
| Coeur | 2,3<br>2,1 - 2,5 | 0,70<br>0,6 - 0,9 | 0,60<br>0,5 - 0,7 | 2,6<br>2,4 - 2,7 | 1,6<br>1,6 - 1,7 | 1,4<br>1,3 - 1,5 | 1,8<br>1,4 - 1,9 | 1,5<br>1,4 - 1,7 | 1,3<br>1,2 - 1,4 |
| Sang total | 3,0<br>2,9 - 3,2 | 2,3<br>2,0 - 2,5 | 3,0<br>2,9 - 3,2 | 9,2<br>7,5 - 10,1 | 2,3<br>1,8 - 3,0 | 1,8<br>1,6 - 2,2 | 3,5<br>2,5 - 4,6 | 2,0<br>1,8 - 2,5 | 1,5<br>1,0 - 1,9 |

**Exemple 10:** Préparation du complexe nitruro-bis(N,N-diméthoxydiéthyl dithiocarbamate) $^{99m}$Tc(V)(TcNMEDC).

a) Préparation du produit intermédiaire.

Dans un flacon type pénicilline, on introduit 0,5ml d'une solution contenant 0,8.$10^{-2}$mol/l (1mg/ml) de S-méthyl N-méthyldithiocarbazate dans l'eau, puis 0,5ml d'une solution à 2.$10^{-2}$mol/l (10mg/ml) de triphénylphosphine trisulfonée dans l'eau et 0,1ml d'acide chlorhydrique 1N. On ajoute ensuite 0,5 à 5ml d'une solution de pertechnétate de sodium ($Tc^{99m}$) et on effectue la réaction à 80°C pendant 30 minutes ou à 100°C pendant 15 minutes.

b) Préparation du complexe final.

Au contenu du flacon obtenu dans l'étape a), on ajoute 0,1ml de solution de NaOH 1N et 0,5ml d'une solution contenant 0,1mol/l de diméthoxyéthyldithiocarbamate de sodium (23mg/ml) dans un tampon carbonate-bicarbonate de sodium à 0,5 mol/l à pH 9,5.

On effectue la réaction pendant 30 minutes à la température ambiante.

On obtient ainsi le complexe nitruro TcNMEDC, soit le complexe de formule (Tc≡N)$L^1L^2$ avec $L^1$ et $L^2$ représentant le composé de formule (VI).

**Exemple 11 :** Préparation du complexe nitruro-bis(N-éthyl,N-(2-méthoxyéthyl)dithiocarbamate)$^{99m}$Tc(V) (TcNEMEC).

On suit le même mode opératoire que dans l'exemple 10 pour préparer le produit intermédiaire à partir de S-méthyl N-méthyldithiocarbazate et de triphénylphosphine trisulfonée. On prépare ensuite le produit final en suivant le même mode opératoire que dans l'exemple 10 mais en utilisant 0,5ml d'une solution à 0,1mol/l de N-éthyl, N-(2-méthoxyéthyl)dithiocarbamate de sodium (20mg/ml) à la place du diméthoxyéthyldithiocarbamate de sodium de l'exemple 10.

On obtient ainsi le complexe nitruro-bis(N-éthyl,N-(2-méthoxyéthyl)dithiocarbamate)$^{99m}$Tc(V), c'est-à-dire le produit de formule :

$$(Tc\equiv N)L^1L^2$$

avec $L^1$ et $L^2$ représentant le composé de formule (VII).

**Exemple 12** : Préparation du complexe nitruro-bis(N-éthyl,N-(3-méthoxypropyl)dithiocarbamate)$^{99m}$Tc(V)-(TcNEMPC).

On suit le même mode opératoire que dans les exemples 10 et 11 pour préparer ce complexe à partir du produit intermédiaire obtenu dans l'étape a) de l'exemple 10 en utilisant comme réactif dans l'étape b) 0,5ml d'une solution à 0,1mol/l de N-éthyl,N(3-méthoxypropyl)dithiocarbamate de sodium (22mg/ml).

On obtient ainsi le complexe nitruro TcNEMPC, soit le complexe de formule (Tc≡N)$L^1L^2$ avec $L^1$ et $L^2$ représentant le composé de formule (VIII).

**Exemple 13** : Préparation du complexe nitruro-bis(N-éthyl,N-(2-éthoxyéthyl)dithiocarbamate)$^{99m}$Tc(V) (TcNEEDC).

On suit le même mode opératoire que dans l'exemple 10 pour préparer le produit intermédiaire et le produit final, sauf que l'on utilise comme réactif pour la préparation du produit final 0,5ml d'une solution à 0,1mol/l de N-éthyl, N(2-éthoxyéthyl)dithiocarbamate de sodium (22mg/ml).

On obtient ainsi le complexe nitruro TcNEEDC, soit le complexe de formule (Tc≡N)$L^1L^2$ avec $L^1$ et $L^2$ représentant le composé de formule (IX).

**Exemple 14 :** Préparation du complexe nitruro-bis(N-méthoxy, N-méthyldithiocarbamate)$^{99m}$Tc(TcNMEMC).

a) Préparation du produit intermédiaire.

On suit le même mode opératoire que dans l'exemple 10 pour préparer un produit intermédiaire en utilisant les mêmes réactifs et les mêmes conditions de réaction.

b) Préparation du complexe final.

Au contenu du flacon obtenu dans l'étape a), on ajoute 0,1ml de solution de NaOH 1N et 0,5ml d'une solution contenant 0,13mol/l de N-méthoxy, N-méthyldithiocarbamate de sodium (20mg/ml) dans un tampon carbonate-bicarbonate de sodium à 0,5mol/l à pH 9,5.

On effectue la réaction pendant 30 minutes à la température ambiante.

On obtient ainsi le complexe TcNMEMC, soit le complexe de formule (Tc≡N)$L^1L^2$ avec $L^1$ et $L^2$ représentant :

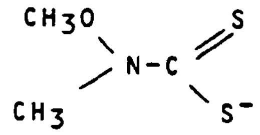

**Exemple 15** : Préparation du complexe nitruro-bis(N-méthoxy, N-éthyldithiocarbamate)$^{99m}$Tc(V) (TcNMEEC).

On suit le même mode opératoire que dans l'exemple 14 pour préparer ce complexe de technétium à partir du même produit intermédiaire en utilisant pour la préparation du produit final 0,5ml d'une solution à 0,12mol/l de N-méthoxy,N-éthyldithiocarbamate de sodium (20mg/ml). On obtient ainsi le complexe TcNMEEC, soit le complexe de formule (Tc≡N)$L^1L^2$ avec $L^1$ et $L^2$ représentant la formule :

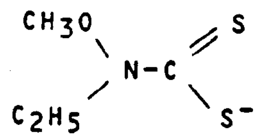

**Exemple 16** : Préparation du complexe nitruro-bis(N-éthoxy,N-méthyldithiocarbamate)$^{99m}$Tc(V)(TcNETMC).

Dans cet exemple, on suit le même mode opératoire que dans l'exemple 14, sauf que l'on utilise comme réactif pour la préparation du produit final, 0,5ml d'une solution à 0,12mol/l de N-éthoxy, N-méthyldithiocarbamate de sodium (20mg/ml).

On obtient ainsi le complexe de technétium de formule (Tc≡N)$L^1L^2$ avec $L^1$ et $L^2$ représentant la formule :

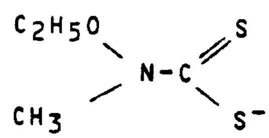

**Exemple 17** : Préparation du complexe nitruro-bis (N-éthoxy,N-éthyldithiocarbamate)$^{99m}$Tc(V) (TcNETEC).

On suit le même mode opératoire que dans l'exemple 14 pour préparer ce complexe de technétium en utilisant comme réactif dans la deuxième étape 0,5ml d'une solution contenant 0,11mol/l de N-éthoxy,N-éthyldithiocarbamate de sodium (20mg/ml).

On obtient ainsi le complexe de formule (Tc≡N)$L^1L^2$ avec $L^1$ et $L^2$ représentant la formule :

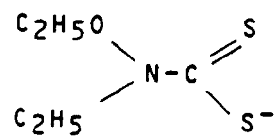

**Exemple 18**.

On teste les propriétés biologiques des complexes obtenus dans les exemples 10 à 13 et 17 en déterminant la captation du myocarde chez des chiens pesant entre 10 et 15kg.

Dans ce cas, on injecte aux chiens anesthésiés avec du pentobarbital de sodium et gardés sous ventilation, une dose correspondant à 2μmol/kg de masse corporelle de complexe de technétium myotrope, ce qui correspond à une dose de rayonnements de 2 à 5mCi.

La captation de la radioactivité par le myocarde et les organes environnants (poumons, foie) est déterminée par acquisition dynamique, entre l'injection et la fin d'examen, à la gamma caméra, en définissant des zones d'intérêt pour chaque organe.

Tous les complexes essayés permettent une bonne visualisation du myocarde.

Les valeurs du contraste coeur/foie et coeur/poumon sont mesurées en effectuant un simple rapport entre les nombres des coups présents par unité de surface (ou pixel) dans les organes.

Les valeurs des rapports coeur/foie, et coeur/poumon sont données dans le tableau 2 annexé.

Les rapports organe cible/bruits de fond sont donc très favorables.

**Exemples 19 à 21.**

Dans ces exemples, on teste les propriétés des complexes obtenus dans les exemples 15 à 18, en déterminant leur biodistribution chez des rats mâles de la race Sprague Dawley, pesant 200±20g.

Dans ce cas, on injecte aux rats, anesthésiés avec du pentobarbital de sodium, une dose correspondant à 15μmol/kg de masse corporelle de ligand myotrope, ce qui correspond à une dose de rayonnement de 1 à 2,5μCi.

5 minutes, 30 minutes, ou 60 minutes après l'injection du produit, on sacrifie les rats, on prélève leurs organes et on détermine la radioactivité présente dans chacun des organes.

Les résultats obtenus sont donnés dans le tableau 3 qui suit, et exprimés en pourcentage de la radioactivité injectée retrouvée dans l'organe, après prélèvement et comptage.

Les valeurs données dans chaque case du tableau représentent la valeur moyenne et les deux valeurs extrêmes.

Au vu de ce tableau, on constate que ces complexes présentent un bon tropisme cardiaque.

## TABLEAU 2

**Rapports coeur/foie, coeur/poumon pour quelques complexes Tc=N dithiocarbamate**

| Ex | $L_1=L_2=R^1R^2>NCS_2$ | Rapports / t min | 5 | 15 | 30 | 75 | 90 | 120 |
|---|---|---|---|---|---|---|---|---|
| 17 | $R^1 = C_2H_5-$ | Coeur/foie | 2,01 | 1,39 | 1,24 | 0,90 | | 0,82 |
| | $R^2 = C_2H_5O-$ | Coeur/poumon | 1,10 | 1,33 | 1,61 | 1,75 | | 2,64 |
| 11 | $R^1 = C_2H_5-$ | Coeur/foie | 1,44 | 1,02 | 0,86 | | 0,66 | |
| | $R^2 = CH_3O(CH_2)_2-$ | Coeur/poumon | 2,75 | 2,60 | 2,41 | | 1,66 | |
| 10 | $R^1 = CH_3O(CH_2)_2-$ | Coeur/foie | 1,27 | 0,61 | 0,38 | | | |
| | $R^2 = CH_3O(CH_2)_2-$ | Coeur/poumon | 2,02 | 1,70 | 1,35 | | | |
| 13 | $R^1 = C_2H_5-$ | Coeur/foie | 1,43 | 0,97 | 0,87 | 0,63 | 0,58 | 0,56 |
| | $R^2 = C_2H_5O(CH_2)_2-$ | Coeur/poumon | 1,36 | 1,66 | 1,68 | 3,10 | 2,97 | 2,46 |
| 12 | $R^1 = C_2H_5-$ | Coeur/foie | 0,95 | 0,74 | 0,60 | 0,32 | 0,27 | 0,18 |
| | $R^2 = CH_3O(CH_2)_3-$ | Coeur/poumon | 1,39 | 2,16 | 2,44 | 2,04 | 1,87 | 1,54 |

# TABLEAU 3

| $L_1 = L_2$ | EX. 6 (TCNMEMC) $CH_3-O$, $CH_3$ >$NCS_2$ | | | EX. 7 (TCNMEEC) $CH_3-O$, $C_2H_5$ >$NCS_2$ | | |
|---|---|---|---|---|---|---|
| Temps entre injection I.V. et sacrifice / organes entiers | 5 min | 30 min | 60 min | 5 min | 30 min | 60 min |
| Foie | 24,2<br>16,1-29,3 | 27,5<br>20-29,2 | 22,7<br>20,5-24,2 | 22,8<br>20,1-23,5 | 21,2<br>20,0-24,0 | 23,0<br>21-25 |
| Reins | 5,8<br>5,0-6,5 | 3,9<br>3,1-4,5 | 5,4<br>4,2-5,9 | 6,0<br>5,8-6,2 | 6,7<br>6,5-7,0 | 8,7<br>7,5-9,1 |
| Poumons | 4,8<br>4,7-4,9 | 6,2<br>5,7-6,5 | 7,8<br>7,0-8,2 | 4,4<br>4,0-4,8 | 3,7<br>3,2-3,9 | 2,5<br>2,1-3,0 |
| Cerveau | 0,46<br>0,36-0,59 | 0,18<br>0,15-0,20 | 0,17<br>0,16-0,20 | 0,40<br>0,40-0,40 | 0,23<br>0,21-0,25 | 0,16<br>0,15-0,1 |
| Coeur | 2,2<br>1,9-2,5 | 0,80<br>0,6-0,9 | 0,20<br>0,2-0,2 | 1,5<br>1,4-1,7 | 0,80<br>0,75-0,83 | 0,50<br>0,48-0,53 |
| Sang total | 2,4<br>2,4-2,4 | 4,5<br>4,2-4,9 | 7,8<br>6,8-8,5 | 7,2<br>7,0-7,5 | 6,1<br>5,8-6,9 | 6,0<br>5,7-6,5 |

**TABLEAU 3 (suite)**

| $L_1 = L_2$ | EX. 8 (TCNETMC) $C_2H_5$-O-N($CH_3$)-$CS_2$ | | | EX. 9 (TCNETEC) $C_2H_5$-O-N($C_2H_5$)-$CS_2$ | | |
|---|---|---|---|---|---|---|
| Temps entre injection I.V. et sacrifice / organes entiers | 5 min | 30 min | 60 min | 5 min | 30 min | 60 min |
| Foie | 18,7<br>17,5-19,1 | 22,1<br>19,5-23,0 | 29,4<br>26,1-32,0 | 21,5<br>16,1-27,2 | 29,4<br>27,5-30,2 | 28,9<br>28,0-29,5 |
| Reins | 5,5<br>5,0-5,9 | 4,0<br>3,8-4,3 | 4,3<br>4,0-4,5 | 6,1<br>5,5-7,4 | 5,7<br>5,0-6,0 | 5,4<br>4,9-5,7 |
| Poumons | 5,3<br>5,0-5,8 | 9,0<br>8,4-9,7 | 6,5<br>6,1-6,9 | 4,9<br>4,8-5,1 | 2,4<br>2,1-2,8 | 1,6<br>1,4-1,8 |
| Cerveau | 0,65<br>0,62-0,69 | 0,18<br>0,15-0,20 | 0,18<br>0,15-0,20 | 0,65<br>0,54-0,75 | 0,34<br>0,30-0,40 | 0,20<br>0,20-0,21 |
| Coeur | 1,70<br>1,60-1,80 | 0,81<br>0,80-0,82 | 0,41<br>0,40-0,42 | 2,70<br>2,32-3,08 | 2,26<br>2,1-2,5 | 1,91<br>1,85-2,01 |
| Sang total | 4,2<br>3,9-4,4 | 3,6<br>3,5-3,7 | 2,4<br>2,3-2,5 | 2,1<br>1,8-3,0 | 2,2<br>2,0-2,2 | 2,3<br>2,0-2,5 |

## Revendications

**Revendications pour les Etats contractants suivants : AT, BE, DE, FR, GB, IT, LU, NL, SE**

**1.** Produit radiopharmaceutique, caractérisé en ce qu'il comprend un complexe d'un métal de transition répondant à la formule :

$$(M \equiv N)\ L^1L^2 \qquad (I)$$

dans laquelle M est un métal de transition et $L^1$ et $L^2$ qui peuvent être identiques ou différents, répondent à la formule :

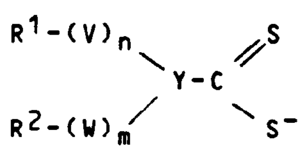

(II)

dans laquelle V et W qui peuvent être identiques ou différents, représentent O, S ou Se, n et m qui peuvent être identiques ou différents, sont égaux à 0 ou à 1, Y représente N, P ou As, et $R^1$ et $R^2$ qui peuvent être identiques ou différents, représentent un radical alkyle linéaire ou ramifié de 1 à 10 atomes de carbone, non substitué ou substitué par des groupements $-O-R^3$, $OOC-R^3$, $OCNR^4R^5$ ou $NR^4R^5$ dans lesquels $R^3$ est un radical alkyle linéaire ou ramifié de 1 à 5 atomes de carbone et $R^4$ et $R^5$ qui peuvent être identiques ou différents, sont des atomes d'hydrogène ou des radicaux alkyle linéaires ou ramifiés de 1 à 5 atomes de carbone, ou dans laquelle $R^1$ et $R^2$ forment ensemble un cycle hydrocarboné contenant éventuellement un ou plusieurs hétéroatomes.

2. Produit radiopharmaceutique selon la revendication 1, caractérisé en ce que M représente un isotope du technétium ou du rhénium.

3. Produit radiopharmaceutique selon la revendication 2, caractérisé en ce que l'isotope de technétium est Tc 99 m.

4. Produit radiopharmaceutique selon la revendication 2, caractérisé en ce que l'isotope de rhénium est Re-186 ou Re-188.

5. Produit radiopharmaceutique selon l'une quelconque des revendications 1 à 4, caractérisé en ce que Y représente N, m et n sont égaux à 0, et $L^1$ et $L^2$ sont identiques.

6. Produit radiopharmaceutique selon la revendication 5, caractérisé en ce que $R^1$ et $R^2$ représentent des radicaux alkyle non substitués.

7. Produit radiopharmaceutique selon la revendication 6, caractérisé en ce que $L^1$ et $L^2$ représentent :

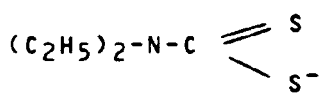

(III)

8. Produit radiopharmaceutique selon la revendication 6, caractérisé en ce que $L^1$ et $L^2$ représentent :

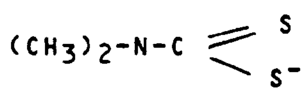

(IV)

9. Produit radiopharmaceutique selon la revendication 6, caractérisé en ce que $L^1$ et $L^2$ représentent :

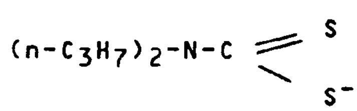

(V)

10. Produit radiopharmaceutique selon la revendication 5, caractérisé en ce que l'un au moins des $R^1$ et $R^2$ est un radical alkoxyalkyle.

11. Produit radiopharmaceutique selon la revendication 10, caractérisé en ce que $R^1$ représente $CH_3O-CH_2-CH_2-$ et $R^2$ représente $CH_3-CH_2-$ ou $CH_3OCH_2-CH_2-$.

12. Produit radiopharmaceutique selon la revendication 10, caractérisé en ce que $R^1$ représente $CH_3-CH_2-$ et $R^2$ représente $CH_3-O-CH_2-CH_2-CH_2-$ ou $C_2H_5-O-CH_2-CH_2-$.

13. Produit radiopharmaceutique selon l'une quelconque des revendications 1 à 4, caractérisé en ce que Y représente N et $L^1$ et $L^2$ répondent à la formule :

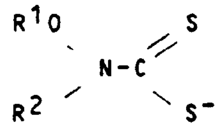

dans laquelle $R^1$ et $R^2$ ont la signification donnée dans la revendication 1.

**14.** Produit radiopharmaceutique selon la revendication 13, caractérisé en ce que $R^1$ représente $CH_3$- et $R^2$ représente $CH_3$- ou $CH_3$-$CH_2$-.

**15.** Produit radiopharmaceutique selon la revendication 13, caractérisé en ce que $R^1$ représente $CH_3$-$CH_2$- et $R^2$ représente $CH_3$- ou $CH_3$-$CH_2$-.

**16.** Procédé de préparation d'un produit radiopharmaceutique selon l'une quelconque des revendications 1 à 15, caractérisé en ce qu'il comprend les étapes successives suivantes :

1°) faire réagir un composé oxygéné d'un métal de transition M avec :

a) un premier ligand choisi dans le groupe des phosphines et polyphosphines aliphatiques et aromatiques, substituées ou non substituées, et

b) un second réactif choisi parmi les azotures de métal alcalin et d'ammonium et les ligands azotés comportant un motif >N-N< dans lesquels les N sont reliés à des atomes d'hydrogène et/ou à des groupements organiques monovalents par l'intermédiaire d'un atome de carbone, ou dans lequel l'un des N est relié à l'atome de carbone d'un groupe organique bivalent par l'intermédiaire d'une double liaison et l'autre N est relié à des atomes d'hydrogène et/ou à des groupements organiques monovalents par l'intermédiaire d'un atome de carbone, et

2°) faire réagir le produit intermédiaire obtenu dans la première étape avec un composé répondant à la formule :

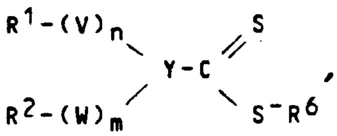

, $pH_2O$ (X)

dans laquelle $R^1$, $R^2$, V, W, n, m et Y ont la signification donnée dans la revendication 1,
$R^6$ est un ion de métal alcalin, $H^+$ ou $NH_4^+$, et p est égal à 0 ou est un nombre entier allant de 1 à 5.

**17.** Procédé selon la revendication 16, caractérisé en ce que le composé oxygéné du métal de transition est un pertechnétate de métal alcalin ou d'ammonium.

**18.** Procédé selon la revendication 16, caractérisé en ce que le composé oxygéné du métal de transition est du perrhénate de métal alcalin ou d'ammonium.

**19.** Procédé selon l'une quelconque des revendications 16 à 18, caractérisé en ce que le premier ligand est une phosphine choisie parmi la triphénylphosphine, la diéthylphénylphosphine, la triéthylphosphine, la triméthylphosphine, la tris(cyanoéthyl-2)phosphine et la triphénylphosphine trisulfonée.

**20.** Procédé selon l'une quelconque des revendications 16 à 19, caractérisé en ce que le second réactif est choisi parmi le S-méthyldithiocarbazate, le S-méthyl-N-méthyldithiocarbazate, l'alpha-N-méthyl-S-méthyl bêta-N-pyridylméthylène dithiocarbazate, le S-méthyl-bêta-N-(2-hydroxyphényl)méthylène dithiocarbazate et le alpha-N-méthyl-S-méthyl-bêta-N-(2-hydroxyphényl)méthylène dithiocarbazate.

**21.** Procédé selon l'une quelconque des revendications 16 à 19, caractérisé en ce que le second réactif est l'azoture de sodium.

**22.** Trousse pour la préparation d'un produit radiopharmaceutique, caractérisée en ce qu'elle comprend :

- un premier flacon contenant une phosphine,
- un second flacon contenant de l'azoture de sodium, de l'acide dithiocarbazique ou un dérivé de celui-ci, et,

- un troisième flacon contenant un composé répondant à la formule :

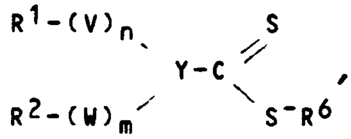
, $pH_2O$ (X)

dans laquelle $R^1$, $R^2$, V, W, n, m et Y ont la signification donnée dans la revendication 1, $R^6$ est un ion de métal alcalin, $H^+$ ou $NH_4^+$, et p est égal à 0 ou est un nombre entier allant de 1 à 5.

**23.** Utilisation du complexe de métal de transition selon l'une quelconque des revendications 1 à 15 pour la fabrication d'un produit radiopharmaceutique à tropisme cardiaque.

**Revendications pour l'Etat contractant suivant : ES**

**1.** Procédé de préparation d'un produit radiopharmaceutique comprenant un complexe d'un métal de transition répondant à la formule :

$$(M = N)\ L^1L^2 \qquad (I)$$

dans laquelle M est un métal de transition et $L^1$ et $L^2$ qui peuvent être identiques ou différents, répondent à la formule :

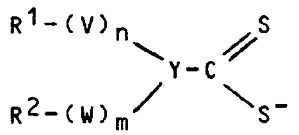
(II)

dans laquelle V et W qui peuvent être identiques ou différents, représentent 0, S ou Se, n et m qui peuvent être identiques ou différents, sont égaux à 0 ou à 1, Y représente N, P ou As, et $R^1$ et $R^2$ qui peuvent être identiques ou différents, représentent un radical alkyle linéaire ou ramifié de 1 à 10 atomes de carbone, non substitué ou substitué par des groupements $-O-R^3$, $OOC-R^3$, $OCNR^4R^5$ ou $NR^4R^5$ dans lesquels $R^3$ est un radical alkyle linéaire ou ramifié de 1 à 5 atomes de carbone et $R^4$ et $R^5$ qui peuvent être identiques ou différents, sont des atomes d'hydrogène ou des radicaux alkyle linéaires ou ramifiés de 1 à 5 atomes de carbone, ou dans laquelle $R^1$ et $R^2$ forment ensemble un cycle hydrocarboné contenant éventuellement un ou plusieurs hétéroatomes, caractérisé en ce qu'il comprend les étapes successives suivantes :

1°) faire réagir un composé oxygéné d'un métal de transition M avec :

a) un premier ligand choisi dans le groupe des phosphines et polyphosphines aliphatiques et aromatiques, substituées ou non substituées, et

b) un second réactif choisi parmi les azotures de métal alcalin et d'ammonium et les ligands azotés comportant un motif >N-N< dans lesquels les N sont reliés à des atomes d'hydrogène et/ou à des groupements organiques monovalents par l'intermédiaire d'un atome de carbone, ou dans lequel l'un des N est relié à l'atome de carbone d'un groupe organique bivalent par l'intermédiaire d'une double liaison et l'autre N est relié à des atomes d'hydrogène et/ou à des groupements organiques monovalents par l'intermédiaire d'un atome de carbone, et

2°) faire réagir le produit intermédiaire obtenu dans la première étape avec un composé répondant à la formule :

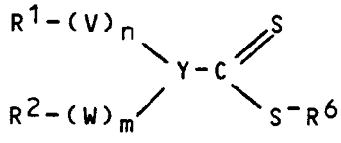
, $pH_2O$ (X)

dans laquelle $R^1$, $R^2$, V, W, n, m et Y ont la signification donnée ci-dessus,
$R^6$ est un ion de métal alcalin, $H^+$ ou $NH_4^+$, et p est égal à 0 ou est un nombre entier allant de 1 à 5.

**2.** Procédé selon la revendication 1, caractérisé en ce que M représente un isotope du technétium ou du

rhénium.

3. Procédé selon la revendication 2, caractérisé en ce que l'isotope de technétium est Tc 99 m.

4. Procédé selon la revendication 2, caractérisé en ce que l'isotope de rhénium est Re-186 ou Re-188.

5. Procédé selon l'une quelconque des revendications 1 à 4, caractérisé en ce que Y représente N, m et n sont égaux à 0, et $L^1$ et $L^2$ sont identiques.

6. Procédé selon la revendication 5, caractérisé en ce que $R^1$ et $R^2$ représentent des radicaux alkyle non substitués.

7. Procédé selon la revendication 6, caractérisé en ce que $L^1$ et $L^2$ représentent :

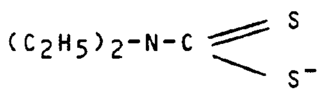

(III)

8. Procédé selon la revendication 6, caractérisé en ce que $L^1$ et $L^2$ représentent :

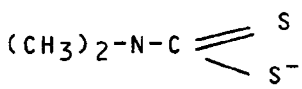

(IV)

9. Procédé selon la revendication 6, caractérisé en ce que $L^1$ et $L^2$ représentent :

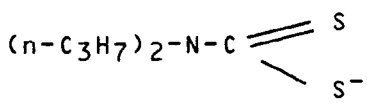

(V)

10. Procédé selon la revendication 5, caractérisé en ce que l'un au moins des $R^1$ et $R^2$ est un radical alkoxyalkyle.

11. Procédé selon la revendication 10, caractérisé en ce que $R^1$ représente $CH_3O\text{-}CH_2\text{-}CH_2\text{-}$ et $R^2$ représente $CH_3\text{-}CH_2\text{-}$ ou $CH_3OCH_2\text{-}CH_2\text{-}$.

12. Procédé selon la revendication 10, caractérisé en ce que $R^1$ représente $CH_3\text{-}CH_2\text{-}$ et $R^2$ représente $CH_3\text{-}O\text{-}CH_2\text{-}CH_2\text{-}CH_2\text{-}$ ou $C_2H_5\text{-}O\text{-}CH_2\text{-}CH_2\text{-}$.

13. Procédé selon l'une quelconque des revendications 1 à 4, caractérisé en ce que Y représente N et $L^1$ et $L^2$ répondent à la formule :

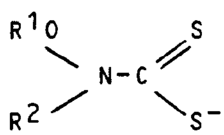

dans laquelle $R^1$ et $R^2$ ont la signification donnée dans la revendication 1.

14. Procédé selon la revendication 13, caractérisé en ce que $R^1$ représente $CH_3\text{-}$ et $R^2$ représente $CH_3\text{-}$ ou $CH_3\text{-}CH_2\text{-}$.

15. Procédé selon la revendication 13, caractérisé en ce que $R^1$ représente $CH_3\text{-}CH_2\text{-}$ et $R^2$ représente $CH_3\text{-}$ ou $CH_3\text{-}CH_2\text{-}$.

16. Procédé selon l'une quelconque des revendications 1 et 5 à 15, caractérisé en ce que le composé oxygéné du métal de transition est un pertechnétate de métal alcalin ou d'ammonium.

17. Procédé selon l'une quelconque des revendications 1 et 5 à 15, caractérisé en ce que le composé oxygéné du métal de transition est du perrhénate de métal alcalin ou d'ammonium.

18. Procédé selon l'une quelconque des revendications 1 à 17, caractérisé en ce que le premier ligand est une phosphine choisie parmi la triphénylphosphine, la diéthylphénylphosphine, la triéthylphosphine, la triméthylphosphine, la tris(cyanoéthyl-2)phosphine et la triphénylphosphine trisulfonée.

19. Procédé selon l'une quelconque des revendications 1 à 18, caractérisé en ce que le second réactif est choisi parmi le S-méthyldithiocarbazate, le S-méthyl-N-méthyldithiocarbazate, l'alpha-N-méthyl-S-méthyl bêta-N-pyridylméthylène dithiocarbazate, le S-méthyl-bêta-N-(2-hydroxyphényl)méthylène dithiocarbazate et le alpha-N-méthyl-S-méthyl-bêta-N-(2-hydroxyphényl)méthylène dithiocarbazate.

20. Procédé selon l'une quelconque des revendications 1 à 18, caractérisé en ce que le second réactif est l'azoture de sodium.

21. Trousse pour la préparation d'un produit radiopharmaceutique, caractérisée en ce qu'elle comprend :
   - un premier flacon contenant une phosphine,
   - un second flacon contenant de l'azoture de sodium, de l'acide dithiocarbazique ou un dérivé de celui-ci, et,
   - un troisième flacon contenant un composé répondant à la formule :

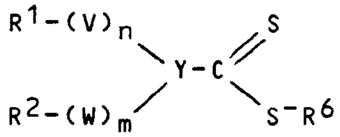, $pH_2O$ (X)

dans laquelle $R^1$, $R^2$, V, W, n, m et Y ont la signification donnée dans la revendication 1, $R^6$ est un ion de métal alcalin, $H^+$ ou $NH_4^+$, et p est égal à 0 ou est un nombre entier allant de 1 à 5.

22. Utilisation du complexe de métal de transition obtenu par le procédé selon l'une quelconque des revendications 1 à 20 pour la fabrication d'un produit radiopharmaceutique à tropisme cardiaque.

## Patentansprüche

### Patentansprüche für folgende Vertragsstaaten : AT, BE, DE, FR, GB, IT, LU, NL, SE

1. Radiopharmazeutisches Produkt, dadurch gekennzeichnet, daß es einen der Formel

$$(M \equiv N)\ L^1L^2 \qquad (I)$$

entsprechenden Übergangsmetallkomplex umfaßt, worin M ein Übergangsmetall ist und $L^1$ und $L^2$, die gleich oder verschieden sein können, der Formel

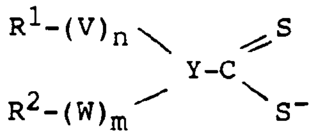 (II)

entsprechen, worin V und W, die gleich oder verschieden sein können, für O, S oder Se stehen, n und m, die gleich oder verschieden sein können, gleich 0 oder gleich 1 sind, Y für N, P oder As steht und $R^1$ und $R^2$, die gleich oder verschieden sein können, einen linearen oder verzweigten Alkylrest mit 1 bis 10 Kohlenstoffatomen bedeuten, der unsubstituiert oder mit Gruppen $-O-R^3$, $OOC-R^3$, $OCNR^4R^5$ oder $NR^4R^5$ substituiert ist, in denen $R^3$ ein linearer oder verzweigter Alkylrest mit 1 bis 5 Kohlenstoffatomen ist und $R^4$ und $R^5$, die gleich oder verschieden sein können, Wasserstoffatome oder lineare oder verzweigte Alkylreste mit 1 bis 5 Kohlenstoffatomen sein können, oder worin $R^1$ und $R^2$ zusammen einen Kohlenwasserstoffring bilden, der gegebenenfalls ein oder mehrere Heteroatome enthält.

2. Radiopharmazeutisches Produkt nach Anspruch 1, dadurch gekennzeichnet, daß M für ein Technetium-

oder Rheniumisotop steht.

3. Radiopharmazeutisches Produkt nach Anspruch 2, dadurch gekennzeichnet, daß das Technetiumisotop Tc-99m ist.

4. Radiopharmazeutisches Produkt nach Anspruch 2, dadurch gekennzeichnet, daß das Rheniumisotop Re-186 oder Re-188 ist.

5. Radiopharmazeutisches Produkt nach einem der Ansprüche 1 bis 4, dadurch gekennzeichnet, daß Y für N steht, m und n gleich 0 sind und $L^1$ und $L^2$ gleich sind.

6. Radiopharmazeutisches Produkt nach Anspruch 5, dadurch gekennzeichnet, daß $R^1$ und $R^2$ unsubstituierte Alkylreste bedeuten.

7. Radiopharmazeutisches Produkt nach Anspruch 6, dadurch gekennzeichnet, daß $L^1$ und $L^2$ für

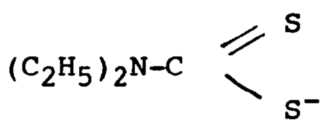

(III)

stehen.

8. Radiopharmazeutisches Produkt nach Anspruch 6, dadurch gekennzeichnet, daß $L^1$ und $L^2$ für

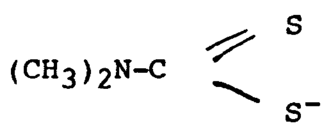

(IV)

stehen.

9. Radiopharmazeutisches Produkt nach Anspruch 6, dadurch gekennzeichnet, daß $L^1$ und $L^2$ für

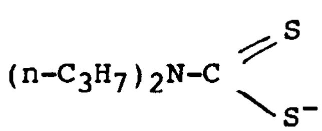

(V)

stehen.

10. Radiopharmazeutisches Produkt nach Anspruch 5, dadurch gekennzeichnet, daß wenigstens einer der Reste $R^1$ und $R^2$ ein Alkoxyalkylrest ist.

11. Radiopharmazeutisches Produkt nach Anspruch 10, dadurch gekennzeichnet, daß $R^1$ für $CH_3O-CH_2-CH_2-$ und $R^2$ für $CH_3-CH_2-$ oder $CH_3OCH_2-CH_2-$ steht.

12. Radiopharmazeutisches Produkt nach Anspruch 10, dadurch gekennzeichnet, daß $R^1$ für $CH_3-CH_2-$ und $R^2$ für $CH_3-O-CH_2-CH_2-CH_2-$ oder $C_2H_5-O-CH_2-CH_2-$ steht.

13. Radiopharmazeutisches Produkt nach einem der Ansprüche 1 bis 4, dadurch gekennzeichnet, daß Y für N steht und $L^1$ und $L^2$ der Formel

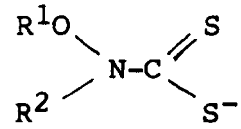

entsprechen, worin $R^1$ und $R^2$ die in Anspruch 1 gegebene Bedeutung haben.

**14.** Radiopharmazeutisches Produkt nach Anspruch 13, dadurch gekennzeichnet, daß $R^1$ für $CH_3$- und $R^2$ für $CH_3$- oder $CH_3$-$CH_2$- steht.

**15.** Radiopharmazeutisches Produkt nach Anspruch 13, dadurch gekennzeichnet, daß $R^1$ für $CH_3$-$CH_2$- und $R^2$ für $CH_3$- oder $CH_3$-$CH_2$- steht.

**16.** Verfahren zur Herstellung eines radiopharmazeutischen Produkts nach einem der Ansprüche 1 bis 15, dadurch gekennzeichnet, daß es nacheinander die folgenden Schritte umfaßt:
1°) eine sauerstoffhaltige Verbindung eines Übergangsmetalls M reagieren zu lassen mit
a) einem ersten Liganden, der in der Gruppe der aliphatiischen und aromatischen, substituierten oder unsubstituierten Phosphine und Polyphosphine gewählt wird, und mit
b) einem zweiten Reagens, das gewählt wird unter den Alkali- und Ammoniumaziden und den stickstoffhaltigen Liganden, die eine Gruppierung >N-N< enthalten, worin die Stickstoffatom über ein Kohlenstoffatom mit Wasserstoffatomen und/oder einwertigen organischen Gruppen verbunden sind oder worin das eine der Stickstoffatome über eine Doppelbindung mit dem Kohlenstoffatom einer zweiwertigen organischen Gruppe und das andere Stickstoffatom über ein Kohlenstoffatom mit Wasserstoffatomen und/oder einwertigen organischen Gruppen verbunden ist, und
2°) das im ersten Schritt erhaltene Zwischenprodukt mit einer der Formel

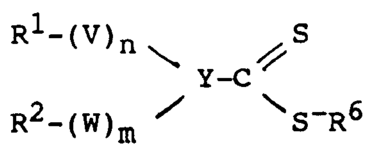 , $pH_2O$ (X)

entsprechenden Verbindung, worin $R^1$, $R^2$, V, W, n, m und Y die in Anspruch 1 gegebene Bedeutung haben, $R^6$ ein Alkalimetallion, $H^+$ oder $NH_4^+$ ist und p gleich 0 oder eine ganze Zahl von 1 bis 5 ist, reagieren zu lassen.

**17.** Verfahren nach Anspruch 16, dadurch gekennzeichnet, daß die sauerstoffhaltige Übergangsmetallverbindung ein Alkali- oder Ammoniumpertechnat ist.

**18.** Verfahren nach Anspruch 16, dadurch gekennzeichnet, daß die sauerstoffhaltige Übergangsmetallverbindung Alkali- oder Ammoniumperrhenat ist.

**19.** Verfahren nach einem der Ansprüche 16 bis 18, dadurch gekennzeichnet, daß der erste Ligand ein unter Triphenylphosphin, Diäthylphenylphosphin, Triäthylphosphin, Trimethylphoshin, Tris(cyanoäthyl-2)phosphin und trisulfoniertem Triphenylphosphin gewähltes Phosphin ist.

**20.** Verfahren nach einem der Ansprüche 16 bis 19, dadurch gekennzeichnet, daß das zweite Reagens unter S-Methyldithiocarbazat, S-Methyl-N-methyldithiocarbazat, α-N-Methyl-S-methyl-β-N-pyridylmethylendithiocarbazat, S-Methyl-β-N-(2-hydroxyphenyl)methylen-dithiocarbazat und α-N-Methyl-S-methyl-ß-N-(2-hydroxyphenyl)methylen-dithiocarbazat gewählt wird.

**21.** Verfahren nach einem der Ansprüche 16 bis 19, dadurch gekennzeichnet, daß das zweite Reagens Natriumazid ist.

**22.** Besteck zur Herstellung eines radiopharmazeutischen Produkts, dadurch gekennzeichnet, daß es
- einen ersten, ein Phosphin enthaltenden Flakon,
- einen zweiten, Natriumazid, Dithiocarbazidsäure oder eines ihrer Derivate enthaltenden Flakon und

- einen dritten Flakon umfaßt, der eine Verbindung enthält, die der Formel

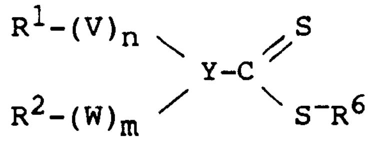

, $pH_2O$ (X)

entspricht, worin $R^1$, $R^2$, V, W, n, m und Y die in Anspruch 1 gegebene Bedeutung haben, $R^6$ ein Alkalimetallion, $H^+$ oder $NH_4^+$ ist und p gleich 0 oder eine ganze Zahl von 1 bis 5 ist.

**23.** Verwendung des Übergangsmetallkomplexes nach einem der Ansprüche 1 bis 15 zur Herstellung eines radiopharmazeutischen Produkts mit Neigung zum Herzgewebe.

**Patentansprüche für folgenden Vertragsstaat : ES**

**1.** Verfahren zur Herstellung eines radiopharmazeutischen Produkts, das einen der Formel

$$(M \equiv N)\ L^1L^2 \qquad (I)$$

entsprechenden Übergangsmetallkomplex umfaßt, worin M ein Übergangsmetall ist und $L^1$ und $L^2$, die gleich oder verschieden sein können, der Formel

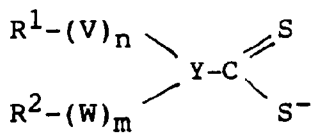

(II)

entsprechen, worin V und W, die gleich oder verschieden sein können, für O, S oder Se stehen, n und m, die gleich oder verschieden sein können, gleich 0 oder gleich 1 sind, Y für N, P oder As steht und $R^1$ und $R^2$, die gleich oder verschieden sein können, einen linearen oder verzweigten Alkylrest mit 1 bis 10 Kohlenstoffatomen bedeuten, der unsubstituiert oder mit Gruppen $-O-R^3$, $OOC-R^3$, $OCNR^4R^5$ oder $NR^4R^5$ substituiert ist, in denen $R^3$ ein linearer oder verzweigter Alkylrest mit 1 bis 5 Kohlenstoffatomen ist und $R^4$ und $R^5$, die gleich oder verschieden sein können, Wasserstoffatome oder lineare oder verzweigte Alkylreste mit 1 bis 5 Kohlenstoffatomen sein können, oder worin $R^1$ und $R^2$ zusammen einen Kohlenwasserstoffring bilden, der gegebenenfalls ein oder mehrere Heteroatome enthält, dadurch gekennzeichnet, daß es nacheinander die folgenden Schritte umfaßt:

1°) eine sauerstoffhaltige Verbindung eines Übergangsmetalls M reagieren zu lassen mit

a) einem ersten Liganden, der in der Gruppe der aliphatiischen und aromatischen, substituierten oder unsubstituierten Phosphine und Polyphosphine gewählt wird, und mit

b) einem zweiten Reagens, das gewählt wird unter den Alkali- und Ammoniumaziden und den stickstoffhaltigen Liganden, die eine Gruppierung ›N-N‹ enthalten, worin die Stickstoffatome über ein Kohlenstoffatom mit Wasserstoffatomen und/oder einwertigen organischen Gruppen verbunden sind oder worin das eine der Stickstoffatome über eine Doppelbindung mit dem Kohlenstoffatom einer zweiwertigen organischen Gruppe und das andere Stickstoffatom über ein Kohlenstoffatom mit Wasserstoffatomen und/oder einwertigen organischen Gruppen verbunden ist, und

2°) das im ersten Schritt erhaltene Zwischenprodukt mit einer der Formel

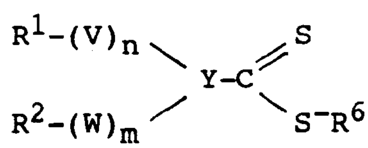

, $pH_2O$ (X)

entsprechenden Verbindung, worin $R^1$, $R^2$, V, W, n, m und Y die oben gegebene Bedeutung haben, $R^6$ ein Alkalimetallion, $H^+$ oder $NH_4^+$ ist und p gleich 0 oder eine ganze Zahl von 1 bis 5 ist, reagieren zu lassen.

**2.** Verfahren nach Anspruch 1, dadurch gekennzeichnet, daß M für ein Technetium- oder Rheniumisotop

steht.

3. Verfahren nach Anspruch 2, dadurch gekennzeichnet, daß das Technetiumisotop Tc-99m ist.

4. Verfahren nach Anspruch 2, dadurch gekennzeichnet, daß das Rheniumisotop Re-186 oder Re-188 ist.

5. Verfahren nach einem der Ansprüche 1 bis 4, dadurch gekennzeichnet, daß Y für N steht, m und n gleich 0 sind und $L^1$ und $L^2$ gleich sind.

6. Verfahren nach Anspruch 5, dadurch gekennzeichnet, daß $R^1$ und $R^2$ unsubtituierte Alkylreste bedeuten.

7. Verfahren nach Anspruch 6, dadurch gekennzeichnet, daß $L^1$ und $L^2$ für

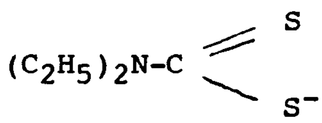

(III)

stehen.

8. Verfahren nach Anspruch 6, dadurch gekennzeichnet, daß $L^1$ und $L^2$ für

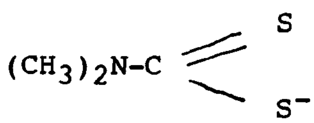

(IV)

stehen.

9. Verfahren nach Anspruch 6, dadurch gekennzeichnet, daß $L^1$ und $L^2$ für

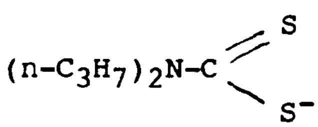

(V)

stehen.

10. Verfahren nach Anspruch 5, dadurch gekennzeichnet, daß wenigstens einer der Reste $R^1$ und $R^2$ ein Alkoxyalkylrest ist.

11. Verfahren nach Anspruch 10, dadurch gekennzeichnet, daß $R^1$ für $CH_3O\text{-}CH_2\text{-}CH_2\text{-}$ und $R^2$ für $CH_3\text{-}CH_2\text{-}$ oder $CH_3OCH_2\text{-}CH_2\text{-}$ steht.

12. Verfahren nach Anspruch 10, dadurch gekennzeichnet, daß $R^1$ für $CH_3\text{-}CH_2\text{-}$ und $R^2$ für $CH_3\text{-}O\text{-}CH_2\text{-}CH_2\text{-}CH_2\text{-}$ oder $C_2H_5\text{-}O\text{-}CH_2\text{-}CH_2\text{-}$ steht.

13. Verfahren nach einem der Ansprüche 1 bis 4, dadurch gekennzeichnet, daß Y für N steht und $L^1$ und $L^2$ der Formel

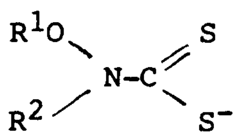

entsprechen, worin $R^1$ und $R^2$ die in Anspruch 1 gegebene Bedeutung haben.

**14.** Verfahren nach Anspruch 13, dadurch gekennzeichnet, daß $R^1$ für $CH_3$- und $R^2$ für $CH_3$- oder $CH_3$-$CH_2$- steht.

**15.** Verfahren nach Anspruch 13, dadurch gekennzeichnet, daß $R^1$ für $CH_3$-$CH_2$- und $R^2$ für $CH_3$- oder $CH_3$-$CH_2$- steht.

**16.** Verfahren nach einem der Ansprüche 1 und 5 bis 15, dadurch gekennzeichnet, daß die sauerstoffhaltige Übergangsmetallverbindung ein Alkali- oder Ammoniumpertechnat ist.

**17.** Verfahren nach einem der Ansprüche 1 und 5 bis 15, dadurch gekennzeichnet, daß die sauerstoffhaltige Übergangsmetallverbindung Alkali- oder Ammoniumperrhenat ist.

**18.** Verfahren nach einem der Ansprüche 1 bis 17, dadurch gekennzeichnet, daß der erste Ligand ein unter Triphenylphosphin, Diäthylphenylphosphin, Triäthylphosphin, Trimethylphoshin, Tris(cyanoäthyl-2)phosphin und trisulfoniertem Triphenylphosphin gewähltes Phosphin ist.

**19.** Verfahren nach einem der Ansprüche 1 bis 18, dadurch gekennzeichnet, daß das zweite Reagens unter S-Methyl-dithiocarbazat, S-Methyl-N-methyldithiocarbazat, α-N-Methyl-S-methyl-β-N-pyridylmethylen-dithiocarbazat, S-Methyl-β-N-(2-hydroxyphenyl)methylen-dithiocarbazat und α-N-Methyl-S-methyl-ß-N-(2-hydroxyphenyl)methylen-dithiocarbazat gewählt wird.

**20.** Verfahren nach einem der Ansprüche 1 bis 18, dadurch gekennzeichnet, daß das zweite Reagens Natriumazid ist.

**21.** Besteck zur Herstellung eines radiopharmazeutischen Produkts, dadurch gekennzeichnet, daß es

- einen ersten, ein Phosphin enthaltenden Flakon,
- einen zweiten, Natriumazid, Dithiocarbazidsäure oder eines ihrer Derivate enthaltenden Flakon und
- einen dritten Flakon umfaßt, der eine Verbindung enthält, die der Formel

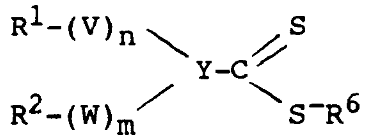 , $pH_2O$ (X)

entspricht, worin $R^1$, $R^2$, V, W, n, m und Y die in Anspruch 1 gegebene Bedeutung haben, $R^6$ ein Alkalimetallion, $H^+$ oder $NH_4^+$ ist und p gleich 0 oder eine ganze Zahl von 1 bis 5 ist.

**22.** Verwendung des durch das Verfahren nach einem der Ansprüche 1 bis 20 erhaltenen Übergangsmetallkomplexes zur Herstellung eines radiopharmazeutischen Produkts mit Neigung zum Herzgewebe.

## Claims

### Claims for the following Contracting States : AT, BE, DE, FR, GB, IT, LU, NL, SE

**1.** Radiopharmaceutical product, characterized in that it comprises a complex of a transition metal complying with the formula:

$$(M \equiv N)\ L^1L^2$$

in which M is a transition metal and $L^1$ and $L^2$, which can be the same or different, comply with the formula:

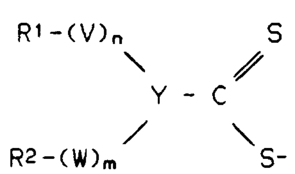 (II)

in which V and W, which can be the same or different, represent O, S or Se, n and m, which can be the

same or different, are equal to 0 or to 1, Y represents N, P or As, and $R^1$ and $R^2$, which can be the same or different, represent a straight or branched alkyl radical with 1 to 10 carbon atoms, which is either not substituted or substituted by $-O-R^3$, $OOC-R^3$, $OCNR^4R^5$ or $-NR^4R^5$ groups, in which $R^3$ is a straight or branched alkyl radical with 1 to 5 carbon atoms and $R^4$ and $R^5$, which can be the same or different, are hydrogen atoms or straight or branched alkyl radicals with 1 to 5 carbon atoms, or in which $R^1$ and $R^2$ together form a hydrocarbon cycle optionally containing one or more heteroatoms.

2. Radiopharmaceutical product according to claim 1, characterized in that M represents a rhenium or technetium isotope.

3. Radiopharmaceutical product according to claim 2, characterized in that the technetium isotope is Tc 99m.

4. Radiopharmaceutical product according to claim 2, characterized in that the rhenium isotope is Re-186 or Re-188.

5. Radiopharmaceutical product according to any one of the claims 1 to 4, characterized in that Y represents N, m and n are equal to 0 and $L^1$ and $L^2$ are identical.

6. Radiopharmaceutical product according to claim 5, characterized in that $R^1$ and $R^2$ represent unsubstituted alkyl radicals.

7. Radiopharmaceutical product according to claim 6, characterized in that $L^1$ and $L^2$ represent:

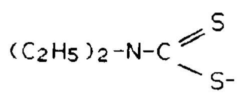

(III)

8. Radiopharmaceutical product according to claim 6, characterized in that $L^1$ and $L^2$ represent:

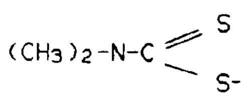

(IV)

9. Radiopharmaceutical product according to claim 6, characterized in that $L^1$ and $L^2$ represent:

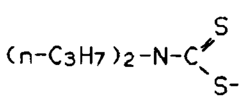

(V)

10. Radiopharmaceutical product according to claim 5, characterized in that at least one of the $R^1$ and $R^2$ is an alkoxyalkyl radical.

11. Radiopharmaceutical product according to claim 10, characterized in that $R^1$ represents $CH_3O-CH_2-CH_2-$ and $R^2$ represents $CH_3-CH_2-$ or $CH_3OCH_2-CH_2-$.

12. Radiopharmaceutical product according to claim 10, characterized in that $R^1$ represents $CH_3-CH_2-$ and $R^2$ represents $CH_3-O-CH_2-CH_2-CH_2-$ or $C_2H_5-O-CH_2-CH_2-$.

13. Radiopharmaceutical product according to any one of the claims 1 to 4, characterized in that Y represents N and $L^1$ and $L^2$ comply with the formula:

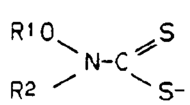

in which $R^1$ and $R^2$ have the meanings given in claim 1.

**14.** Radiopharmaceutical product according to claim 13, characterized in that $R^1$ represents $CH_3$- and $R^2$ represents $CH_3$- or $CH_3$-$CH_2$-.

**15.** Radiopharmaceutical product according to claim 13, characterized in that $R^1$ represents $CH_3$-$CH_2$- and $R^2$ represents $CH_3$- or $CH_3$-$CH_2$-.

**16.** Process for the preparation of a radiopharmaceutical product according to any one of the claims 1 to 15, characterized in that it comprises the following successive stages:

1°) reacting an oxygenated compound of a transition metal M with:

a) a first ligand chosen from within the group of substituted or unsubstituted, aromatic and aliphatic phosphines and polyphosphines and

b) a second reagent chosen from among the ammonium and alkali metal nitrides and the nitrogenous ligands having a >N-N< in which the N are connected to hydrogen atoms and/or to monovalent organic groups via a carbon atom, or in which one of the N is connected to the carbon atom of a divalent organic group via a double bond and the other N is connected to hydrogen atoms and/or monovalent organic groups via a carbon atom and

2°) reacting the intermediate obtained in the first stage with a compound in accordance with the formula:

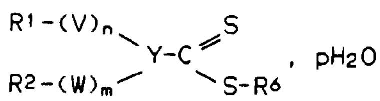

(X)

in which $R^1$, $R^2$, V, W, n, m and Y have the meanings given in claim 1, $R^6$ is an alkali metal ion, $H^+$ or $NH_4^+$ and p is equal to 0 or an integer between 1 and 5.

**17.** Process according to claim 16, characterized in that the oxygenated compound of the transition metal is an ammonium or alkali metal pertechnetate.

**18.** Process according to claim 16, characterized in that the oxygenated compound of the transition metal is ammonium or alkali metal perrhenate.

**19.** Process according to any one of the claims 16 to 18, characterized in that the first ligand is a phosphine chosen from among triphenyl phosphine, diethyl phenyl phosphine, triethyl phosphine, trimethyl phosphine, tris(2-cyanoethyl)-phosphine and trisulphonated triphenyl phosphine.

**20.** Process according to any one of the claims 16 to 19, characterized in that the second reagent is chosen from among S-methyldithiocarbazate, S-methyl-N-methyldithiocarbazate, alpha-N-methyl-S-methyl beta-N-pyridylmethylene dithiocarbazate, S-methyl-beta-N-(2-hydroxyphenyl)methylene dithiocarbazate and alpha-N-methyl-S-methyl-beta-N-(2-hydroxyphenyl)methylene dithiocarbazate.

**21.** Process according to any one of the claims 16 to 19, characterized in that the second reagent is sodium nitride.

**22.** Kit for the preparation of a radiopharmaceutical product, characterized in that it comprises a first bottle containing a phosphine, a second bottle containing sodium nitride, dithiocarbazic acid or a derivative thereof and a third bottle containing a compound complying with the formula:

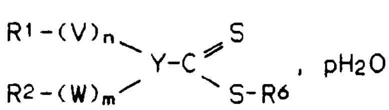

(X)

in which $R^1$, $R^2$, V, W, n, m and Y have the meanings given in in claim 1, $R^6$ is an alkali metal ion, $H^+$ or $NH_4^+$ and p is equal to 0 or an integer from 1 to 5.

23. Use of the transition metal complex according to any one of the claims 1 to 15 for the preparation of a radiopharmaceutical product with cardiac tropism.

## Claims

### Claims for the following Contracting State : ES

1. Process for the preparation of a radiopharmaceutical product comprising a complex of a transition metal complying with the formula:

$$(M = N)\ L^1L^2 \qquad (I)$$

in which M is a transition metal and $L^1$ and $L^2$, which can be the same or different, comply with the formula:

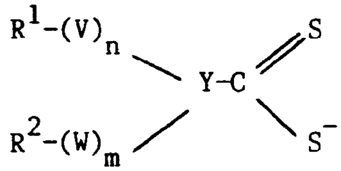

(II)

in which V and W, which can be the same or different, represent O, S or Se, n and m, which can be the same or different, are equal to 0 or to 1, Y reprsents N, P or As, and $R^1$ and $R^2$, which can be the same or different, represent a straight or branched alkyl radical with 1 to 10 carbon atoms, substituted or not by -O-$R^3$, OOC-$R^3$, OCN$R^4R^5$ or N$R^4R^5$ groups, in which $R^3$ is a straight or branched alkyl radical with 1 to 5 carbon atoms and $R^4$ and $R^5$, which can be the same or different, are hydrogen atoms or straight or branched alkyl radicals with 1 to 5 carbon atoms or in which $R^1$ and $R^2$ together form a hydrocarbon cycle optionally containing one or more heteroatoms, characterized in that it comprises the following successive stages:

1) reacting an oxygenated compound of a transition metal M with:

a) a first ligand chosen from within the group of substituted or unsubstituted, aromatic and aliphatic phosphines and polyphosphines and

b) a second reagent chosen from among the ammonium and alkali metal nitrides and the nitrogenous ligands having a >N-N< in which the N are connected to hydrogen atoms and/or to monovalent organic groups via a carbon atom, or in which one of the N is connected to the carbon atom of a divalent organic group via a double bond and the other N is connected to hydrogen atoms and/or monovalent organic groups via a carbon atom and

2) reacting the intermediate obtained in the first stage with a compound in accordance with the formula:

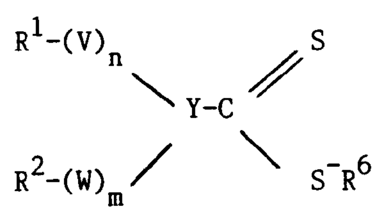

$pH_2O$ (X)

in which $R^1$,$R^2$, V, W, n, m and Y have the meanings given hereinbefore, $R^6$ is an alkali metal ion, $H^+$ or $NH_4^+$ and p is equal to 0 or an integer between 1 and 5.

2. Process according to claim 1, characterized in that M represents a rhenium or technetium isotope.

3. Process according to claim 2, characterized in that the technetium isotope is Tc 99 m.

4. Process according to claim 2, characterized in that the rhenium isotope is Re-186 or Re-188.

5. Process according to any one of the claims 1 to 4, characterized in that Y represents N, m and n are equal to 0 and $L^1$ and $L^2$ are identical.

**6.** Process according to claim 5, characterized in that $R^1$ and $R^2$ represent unsubstituted alkyl radicals.

**7.** Process according to claim 6, characterized in that $L^1$ and $L^2$ represent:

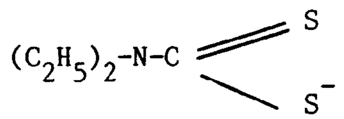
(III)

**8.** Process according to claim 6, characterized in that $L^1$ and $L^2$ represent:

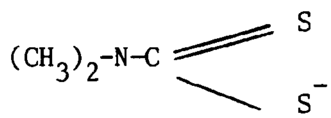
(IV)

**9.** Process according to claim 6, characterized in that $L^1$ and $L^2$ represent:

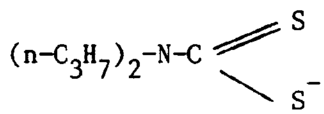
(V)

**10.** Process according to claim 5, characterized in that at least one of the $R^1$ and $R^2$ is an alkoxyalkyl radical.

**11.** Process according to claim 10, characterized in that $R^1$ represents $CH_3O-CH_2-CH_2-$ and $R^2$ represents $CH_3-CH_2-$ or $CH_3OCH_2-CH_2-$.

**12.** Process according to claim 10, characterized in that $R^1$ represents $CH_3-CH_2-$ and $R^2$ represents $CH_3-O-CH_2-CH_2-CH_2-$ or $C_2H_5-O-CH_2-CH_2-$.

**13.** Process according to any one of the claims 1 to 4, characterized in that Y represents N and $L^1$ and $L^2$ comply with the formula:

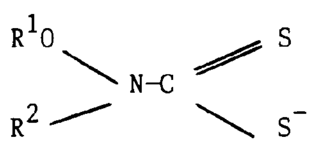

in which $R^1$ and $R^2$ have the meanings given in claim 1.

**14.** Process according to claim 13, characterized in that $R^1$ represents $CH_3-$ and $R^2$ represents $CH_3-$ or $CH_3-CH_2-$.

**15.** Process according to claim 13, characterized in that $R^1$ represents $CH_3-CH_2-$ and $R^2$ represents $CH_3-$ or $CH_3-CH_2-$.

**16.** Process according to any one of the claims 1 and 5 to 15, characterized in that the oxygenated compound of the transition metal is an alkali metal or ammonium pertechnetate.

**17.** Process according to any one of the claims 1 and 5 to 15, characterized in that the oxygenated compound of the transition metal is alkali metal or ammonium perrhenate.

**18.** Process according to any one of the claims 1 to 17, characterized in that the first ligand is a phosphine chosen from among triphenyl phosphine, diethyl phenyl phosphine, triethyl phosphine, trimethyl phosphine, tris-(2-cyanoethyl)-phosphine and trisulphonated triphenyl phosphine.

**19.** Process according to any one of the claims 1 to 18, characterized in that the second reagent is chosen

from among S-methyl dithiocarbazate, S-methyl-N-methyl dithiocarbazate, alpha-N-methyl-S-methyl-beta-N-pyridyl methylene dithiocarbazate, S-methyl-beta-N-(2-hydroxyphenyl)-methylene dithiocarbazate and alpha-N-methyl-S-methyl-beta-N-(2-hydroxyphenyl)-methylene dithiocarbazate.

**20.** Process according to any one of the claims 1 to 18, characterized in that the second reagent is sodium nitride.

**21.** Kit for the preparation of a radiopharmaceutical product, characterized in that it comprises a first bottle containing a phosphine, a second bottle containing sodium nitride, dithiocarbazic acid or a derivative thereof and a third bottle containing a compound complying with the formula:

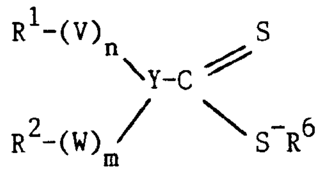

$pH_2O$ (X)

in which $R^1$, $R^2$, V, W, n, m and Y have the meanings given in claim 1, $R^6$ is an alkali metal ion, $H^+$ or $NH_4^+$ and p is equal to 0 or an integer from 1 to 5.

**22.** Use of the transition metal complex obtained by the process according to any one of the claims 1 to 20 for producing a radiopharmaceutical product with cardiac tropism.